# FASCICULE DE BREVET EUROPEEN

(11) **EP 3 443 322 B1**
(45) Date de publication et mention de la délivrance du brevet: **01.01.2020**
(21) Numéro de dépôt: 17717445.5
(22) Date de dépôt: 14.04.2017
(51) Int. Cl.: G01N 15/06, G01N 15/14, B01L 3/00, C12Q 1/6804, G01N 33/50, G01N 15/00

(54) **PROCÉDÉ ET SYSTÈME DE RÉCUPÉRATION DE PRODUITS D'UNE ÉMULSION**
VERFAHREN UND SYSTEM ZUR RÜCKGEWINNUNG VON PRODUKTEN AUS EINER EMULSION
METHOD AND SYSTEM FOR THE RECOVERY OF PRODUCTS FROM AN EMULSION

(30) Priorité: 15.04.2016 FR 1653381
(43) Date de publication de la demande: 20.02.2019
(73) Titulaire: Ecole Supérieure de Physique et de Chimie Industrielles de la Ville de Paris, 75005 Paris (FR)
(72) Inventeur: BAUDRY, Jean, 75011 Paris (FR); BIBETTE, Jérôme, 75005 Paris (FR); BOITARD, Laurent, 75019 Paris (FR); EYER, Klaus, 75019 Paris (FR); LANGER, Krzysztof, 194 63 Upplands Vasby (SE)
(74) Mandataire: Lavoix
(86) Numéro de dépôt international: PCT/EP2017/059070
(87) Numéro de publication internationale: WO 2017/178652

(56) Documents cités:
- WO-A1-2009/025802
- WO-A2-2015/015198
- JP-A- 2015 142 586
- US-A1- 2010 055 677

## Description

La présente invention concerne un procédé de sélection et de récupération de produit. Un tel procédé est destiné, par exemple, à récupérer un produit particulier contenu dans certaines gouttes d'une émulsion. Par exemple le produit peut résulter d'une réaction chimique ou d'une réaction biologique ayant eu lieu dans la goutte.

Il est connu d'utiliser des systèmes de cytométrie en flux pour caractériser des particules isolées, notamment des cellules, entraînées par un flux liquide ou gazeux, avant de les trier. Cependant, de tels systèmes nécessitent un défilement des particules à trier et permettent simplement d'effectuer des mesures simples avant la récupération. Ces systèmes sont utilisés notamment pour trier des cellules présentant des marqueurs membranaires ou intracellulaires. Cependant, ils ne permettent pas de trier les cellules selon les molécules qu'elles sécrètent. En outre, ces systèmes ne permettent pas d'effectuer plusieurs mesures successivement. Ainsi, ces systèmes sont mal adaptés à la caractérisation de produits par suivi de réactions cinétiques. On ne peut donc pas utiliser de tels systèmes pour sélectionner des particules ou des gouttes en se fondant sur des paramètres multiples, comme dans le cas de sélection de cellules selon des phénotypes complexes.

JP 2015 142586 A décrit un procédé comprenant un tri de gouttes.

Le document WO 2010/007307 A2 décrit un procédé de lecture d'une émulsion dans une chambre, permettant d'obtenir des informations riches sur le contenu des gouttes. Après la mesure, une goutte spécifique, présentant des caractères particuliers, peut être récupérée, individuellement, par prélèvement au moyen d'une micropipette aspirant la goutte spécifique.

Cependant, les gouttes spécifiques étant disséminées en différents points de l'émulsion, leur récupération est laborieuse et difficilement automatisable. En outre, l'aspiration d'une goutte de l'émulsion par la micropipette nécessite le retrait de la paroi supérieure de la chambre contenant l'émulsion. Cette étape engendre des risques d'évaporation et de contamination des gouttes pouvant aboutir à une détérioration du produit à récupérer.

Un but de l'invention est de fournir un procédé de sélection et de récupération de produits plus fiable et plus rapide que les procédés existants, permettant une caractérisation précise et une récupération efficace du contenu intéressant d'une goutte.

A cet effet, l'invention a pour objet un procédé de sélection et de récupération de produits selon la revendication 1.

Le procédé selon l'invention peut comprendre l'une ou plusieurs des caractéristiques des revendications 2 à 8 ou la caractéristique suivante, prises isolément ou suivant toute combinaison techniquement possible.

L'invention a également pour objet un système de sélection et de récupération de produits selon la revendication 9.

L'invention sera mieux comprise à la lecture de la description qui va suivre, donnée uniquement à titre d'exemple, et faite en se référant aux dessins annexés, sur lesquels :
- la figure 1 est une représentation schématique d'un système de sélection et de récupération de produits,
- la figure 2 est une représentation schématique d'une émulsion avant l'injection dans le système de récupération des gouttes,
- les figures 3 à 4 illustrent un premier exemple d'application du système de sélection et de récupération de produits,
- la figure 5 illustre un deuxième exemple d'application du système de sélection et de récupération de produits,
- la figure 6 illustre un troisième exemple d'application du système de sélection et de récupération de produits,
- la figure 7 illustre un quatrième exemple d'application du système de sélection et de récupération de produits,
- les figures 8 et 9 illustrent un cinquième exemple d'application du système de sélection et de récupération de produits,
- les figures 10 et 11 illustrent un sixième exemple d'application du système de sélection et de récupération de produits, et
- la figure 12 illustre un septième exemple d'application du système de sélection et de récupération de produits.

Dans la description qui va suivre, les termes « amont » et « aval » et les termes « entrée » et « sortie » sont utilisées en référence au sens normaux de circulation des fluides du système. Le terme « longitudinal » est défini par rapport à la direction du conduit de circulation dans la puce. On appelle « plan transversal » les plans qui sont perpendiculaires à la direction longitudinale. Le terme « diamètre » d'un élément désigne l'étendue transversale maximale de l'élément considérée dans un plan transversal.

Dans la suite de la description, le terme « cellule » désigne une cellule eucaryote ou procaryote. Les cellules sont, par exemple, des bactéries, des levures, des algues, des champignons, des cellules de mammifères ou autres.

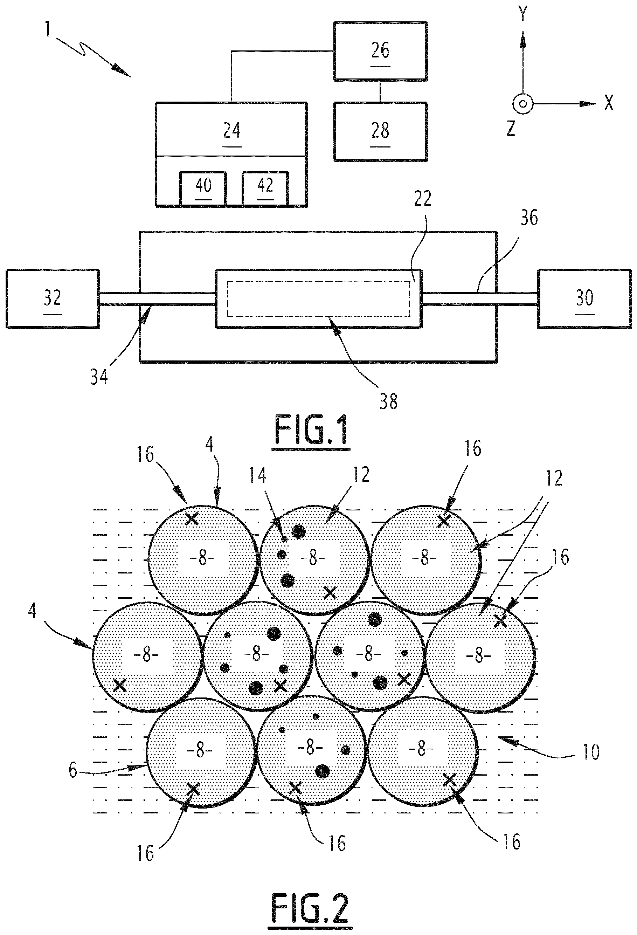

Un système 1 de sélection et de récupération de produits est représenté sur la figure 1. En référence à la figure 2, le système de récupération 1 de gouttes est prévu pour analyser les gouttes 4 d'une émulsion 6, et récupérer sélectivement des produits contenus dans certaines gouttes 4.

Une émulsion 6 de gouttes 4 est représentée sur la figure 2. L'émulsion 6 est constituée d'une pluralité de gouttes 4 d'un fluide interne 8 dispersées dans un fluide porteur 10. Chaque goutte 4 constitue un compartiment fermé rempli de fluide interne 8 dans lequel peut, par exemple, se produire des réactions chimiques ou biologiques pouvant aboutir à la mise en évidence, à la formation ou à la modification d'un produit.

L'émulsion 6 est sensiblement stable, cela signifie que pour un volume fixe de l'émulsion 6, le nombre et le volume des gouttes 4 varient de moins de 5 % lorsque le volume fixe de l'émulsion 6 est conservé entre -80 °C et 80 °C, à 1 bar pendant 48 h. L'émulsion 6 est concentrée. Ceci signifie que la fraction volumique de gouttes 4 dans l'émulsion 6 est comprise entre 5 % et 99,9 %, avantageusement entre 50 % et 80 %, notamment égale à 74 %. Les gouttes 4 de l'émulsion 6 sont, de préférence, sensiblement monodisperses, cela signifie que la polydispersité est inférieure à 5%. Les gouttes 4 présentent, par exemple, un volume compris entre 1 pL et 2 µL. Avantageusement, le volume des gouttes 4 est compris entre 20 pL et 50 pL, notamment dans les applications d'analyse cellulaire.

Ainsi, le diamètre des gouttes est généralement compris entre 32 micromètres et 46 micromètres.

Au moins certaines gouttes 4 de l'émulsion 6 sont différentes d'autres gouttes 4 de l'émulsion 6. Chaque goutte 4 comprend ainsi un fluide interne 8 potentiellement différent d'une goutte 4 à l'autre. Avantageusement, le fluide interne 8 de toutes les gouttes 4 comprend au moins une même base commune 12.

Le fluide interne 8 de chaque goutte 4 est constitué d'éléments propres 14 à la goutte 4 et de la base commune 12. Les proportions des éléments propres 14 et de la base commune 12 et/ou les natures des éléments propres 14 varient d'une goutte 4 à l'autre.

Par exemple, la base commune 12 comprend une solution tampon adaptée à la survie de cellules comme une solution de tampon phosphate salin ou un milieu de culture. Avantageusement, plus de 10 % du volume de la goutte 4 est constitué de la base commune 12.

Avantageusement, la base commune 12 de chaque goutte 4 comprend un réactif de signalisation 16. Le réactif de signalisation 16 est présent dans les mêmes proportions dans chaque goutte 4. Le réactif de signalisation 16 est propre à être activé par un dispositif d'étiquetage 28 comme cela sera décrit ultérieurement.

Par exemple, le réactif de signalisation 16 est propre à émettre un signal de fluorescence seulement après son activation. Avantageusement, l'intensité du signal de fluorescence émis par le réactif de signalisation 16 dépend des conditions de l'activation.

Par exemple, le réactif de signalisation 16 comprend de la fluorescéine en cage de CMNB (5-carboxymethoxy-2-nitrobenzyl) commercialisé par ThermoFisher, sous l'appelation « Fluorescein bis-(5-carboxymethoxy-2-nitrobenzyl) ether, dipotassium salt (CMNB-caged fluorescein) ». Ce composé est incolore est non fluorescent avant sa photolyse. Le groupe formant la cage est propre être lysé sous des flashs ultraviolet (UV) à 355 nm de sorte permettre la libération de la fluorescéine. La fluorescéine est une molécule fortement fluorescente qui peut servir d'étiquetage.

Les éléments propres 14 d'une goutte 4 sont, par exemple, des réactifs chimiques ou biologiques propre à réagir entre eux, ou des produits d'une réaction. Par exemple, les éléments propres 14 d'une goutte 4 sont une cellule et des éléments secrétés par la cellule, comme des protéines.

Par exemple, un élément propre 14 de certaines gouttes 4 est un produit fluorescent avec une longueur d'excitation et d'émission connue mais dont les proportions sont inconnues.

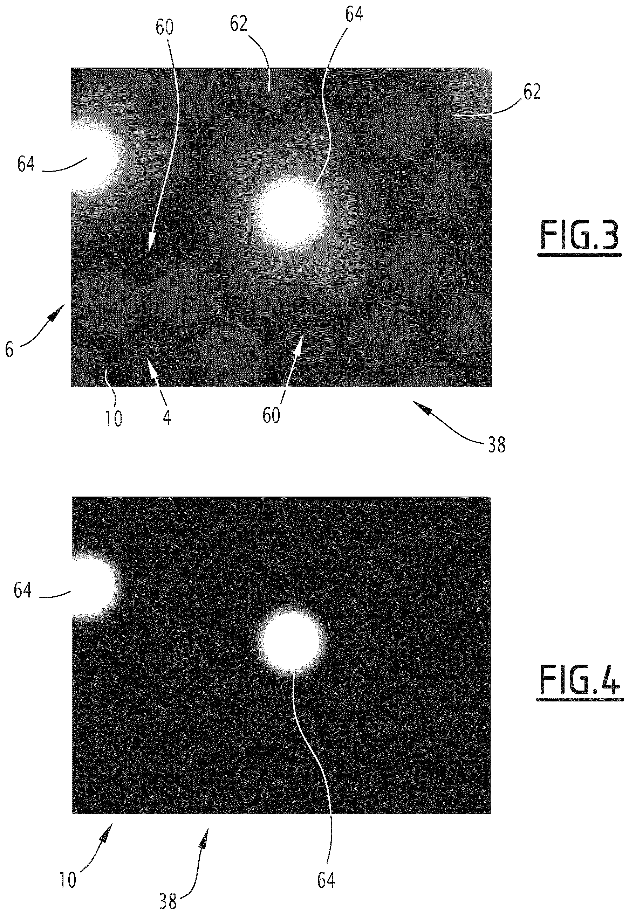

Dans un premier exemple, illustré par les figures 3 et 4, la concentration d'un même élément propre 14 fluorescent, la sulforhodamine, varie d'une goutte 4 à l'autre comme cela sera décrit par la suite.

Le fluide interne 8 de chaque goutte 4 est non miscible avec le fluide porteur 10. On entend par non miscible que le coefficient de partage entre les deux fluides est inférieur à 10⁻³. Les gouttes 4 sont bien définies dans l'émulsion 6 et les échanges entre deux gouttes 4 voisines dans l'émulsion 6 sont limités aux composés solubles ou légèrement solubles dans le fluide porteur 10, c'est-à-dire avec un coefficient de partage supérieur ou égal à 10⁻³ et dans les cas où les compositions sont différentes entre gouttes 4 voisines. Par exemple, le fluide interne 8 est une phase aqueuse et le fluide porteur 10 est une phase organique notamment une phase huileuse. Le fluide porteur 10 comprend par exemple des hydrofluoroéthers comme FC-40 ou HFE-7500, formant une huile fluorée.

Le fluide porteur 10 comprend, en outre, avantageusement un tensio-actif. Le tensio-actif est propre à stabiliser l'émulsion 6. Le tensio-actif est, par exemple, un copolymère à bloc de polyéthylène glycol et de perfluoropolyéther (PEG-PFPE), de Krytox-Di-PEG obtenu par Ran Biotechnolgies ou de QX200™ Droplet Generation Oil with surfactant obtenu par BioRad. Par exemple, la concentration de tensio-actif dans le fluide porteur 10 est comprise entre 2 % et 5 %.

Comme illustré sur la figure 1, le système 1 comprend un support 20 définissant une chambre 22 pour recevoir une émulsion 6. Le système 1 comprend, en outre, un dispositif de mesure 24 d'au moins un paramètre physique pour plusieurs gouttes 4 de l'émulsion 6 reçues dans le support 20, une unité de classement 26, un dispositif d'étiquetage 28, et un dispositif de récupération 30.

L'émulsion 6 est, par exemple, préparée par un dispositif de préparation. Elle est conservée avant d'être placée dans la chambre du système 1 par un module d'injection 32 de l'émulsion. Par exemple, le module d'injection 32 comprend un pousse-seringue, un dispositif piézoélectrique ou un contrôleur de flux basé sur la pression.

Par exemple, le support 20 est une puce. La puce 20 est, dans l'exemple, un bloc rectangulaire étendu selon l'axe longitudinal X et un axe transversal Y perpendiculaire à l'axe longitudinal X. En outre, la puce présente une épaisseur selon un axe d'élévation Z perpendiculaire à l'axe longitudinal X et à l'axe transversal Y. La puce 20 comprend un conduit d'entrée 34, la chambre 22 et un conduit de sortie 36. Le conduit d'entrée 34 est propre à être mis en communication fluidique en amont avec le module d'injection 32 de l'émulsion. Le conduit de sortie 36 est propre à être mis en communication fluidique en aval avec le dispositif de récupération 30, comme illustré sur la figure 1.

La chambre 22 est destinée à stocker une pluralité de gouttes 4 dans le fluide porteur 10 pendant une étape de mesure et d'étiquetage. La chambre 22 présente avantageusement des dimensions adaptée aux dimensions de l'émulsion 6 à étudier pour que les gouttes 4 soient réparties dans la chambre après une étape de chargement de l'émulsion 6. Les gouttes 4 sont avantageusement réparties en une couche bidimensionnelle selon le plan de la puce comprenant l'axe longitudinal X et à l'axe transversal Y. Aucune goutte 4 ne se superpose à une autre goutte 4.

Par exemple, les gouttes 4 sont sphériques dans la chambre 22 et la hauteur de la chambre 22 selon l'axe Z est sensiblement égale au diamètre d'une goutte 4. En variante, la hauteur de la chambre 22 est inférieure au diamètre des gouttes 4 de l'émulsion 6 et les gouttes 4 ont la forme de palets aplatis selon l'axe Z dans la chambre 22.

La chambre 22 présente une région de mesure 38. La région de mesure 38 est propre à contenir entre 1 000 gouttes et 10 000 000 gouttes de l'émulsion simultanément, notamment 10⁵ gouttes. En outre, les dimensions de la région de mesure 38 sont adaptées pour qu'elle puisse être entièrement balayée par le dispositif de mesure 24 et par le dispositif d'étiquetage 28.

La puce 20 est transparente au moins dans la région de mesure 38 pour permettre la mesure d'un paramètre physique sur les gouttes 4 présentes dans la région de mesure 38 par le dispositif de mesure 24.

Avantageusement la puce 20 est réalisée en matériau transparent, par exemple en verre ou des plastiques transparents comme le polydiméthylsiloxane (PDMS), le polyméthacrylate de méthyle (PMMA), des copolymères de cyclooléfines (COC), ou similaire. Le matériau de la puce 20 est avantageusement imperméable au fluide porteur 10.

Successivement ou simultanément, le dispositif de mesure 24 est propre à mesurer au moins un paramètre physique pour plusieurs gouttes 4 de l'émulsion 6 reçue dans le support 20. Avantageusement, il est propre à mesurer le paramètre pour toutes les gouttes 4 présentes dans la région de mesure 38.

Le dispositif de mesure 24 permet notamment une mesure optique des gouttes 4.

Par exemple, le dispositif de mesure 24 comprend un capteur 40 propre à détecter la lumière provenant d'une goutte 4 de la région de mesure 38 et une source de lumière 42.

Par exemple, le capteur 40 est une caméra CCD propre à former une image de la région de mesure 38. La résolution d'image obtenue permet par exemple d'identifier des types cellulaires présents dans les gouttes 4 par des critères morphologiques.

La source de lumière est, par exemple, apte à éclairer sélectivement une goutte 4 sans éclairer la goutte 4 voisine.

En variante ou en complément, la source de lumière 42 est apte à éclairer la zone de mesure 38 entièrement.

Par exemple, la source de lumière 42 est une source de lumière blanche agencée pour éclairer les gouttes 4 de la région de mesure 38. En variante ou en complément, la source de lumière 42 comprend un laser d'excitation ou une lampe fluorescente agencés pour émettre un rayonnement sur la région de mesure 38.

Avantageusement, le capteur 40 et la source 42 du dispositif de mesure 24 permettent des mesures sur plusieurs canaux de fluorescence, à l'échelle de chaque goutte. Par exemple, les combinaisons suivantes ont été utilisées pour le dispositif de mesure 24 : un ensemble de filtre GFP (excitation 465-495 nm, dichroïque 505 nm, émission 515-555 nm), un filtre TRITC (excitation 530-560 nm, dichroïque 570 nm, émission 590-650 nm) et filtre Cy5 (excitation 600-650 nm, dichroïque 665 nm, émission 670-725 nm). Chaque canal de fluorescence peut servir à la mesure de différentes caractéristiques.

Le dispositif de mesure 24 est par exemple un dispositif à balayage bidimensionnel tel que décrit dans le document WO 2010/007307 A2.

En outre le dispositif de mesure 24 est propre à communiquer les mesures obtenues à l'unité de classement 26.

L'unité de classement 26 est propre à recevoir les signaux du dispositif de mesure 24 et à les traiter et à enregistrer les caractéristiques des gouttes 4. Par exemple, l'unité de classement 26 comporte une mémoire et un microprocesseur. L'unité de classement 26 est propre à décider du classement d'au moins une goutte 4 de l'émulsion 6 en fonction des mesures obtenues lors de l'étape de mesure.

Des critères de classement sont, par exemple enregistrés, dans la mémoire. En variante ou en complément, la mémoire est propre à enregistrer des mesures successives effectuées à différents instants par le dispositif de mesure 24, par exemple, pour un suivi cinétique de réaction au sein de plusieurs gouttes 4. Le microprocesseur est propre à effectuer des calculs à partir des mesures et à comparer les résultats des calculs à des paramètres aux critères de classement.

Avantageusement, l'unité de classement 26 comporte une interface homme machine pour qu'un utilisateur puisse récupérer les résultats obtenus lors de l'étape de mesure et ajuster les critères de classement en fonction de ces souhaits ou des résultats obtenus lors de l'étape de mesure.

Le dispositif d'étiquetage 28 est propre à étiqueter une goutte 4 ou une partie de la goutte 4 en fonction de la décision de classement de la goutte 4.

Par exemple, le dispositif d'étiquetage 28 comprend un laser propre à éclairer spécifiquement une goutte 4 avec des paramètres déterminés de sorte à activer le réactif de signalisation 16.

Dans un exemple, on utilise deux sources de lumière distinctes pour le dispositif d'étiquetage 28 et pour le dispositif de mesure 24.

Dans une variante, la même source de lumière 42 sert à la fois pour le dispositif de mesure 24 et pour le dispositif d'étiquetage 28. Par exemple, au cours de l'étape de mesure, la source de lumière 42 éclaire l'ensemble de la région de mesure 38 pour avoir un débit de lecture important. Mais lors de l'étape d'étiquetage, la source de lumière 42 éclaire les gouttes 4 sélectivement pour ne pas étiqueter deux gouttes 4 voisines.

En variante, le dispositif d'étiquetage 28 comprend une source propre à chauffer spécifiquement une goutte 4 avec des paramètres déterminés de sorte à activer le réactif de signalisation 16.

En variante, le dispositif d'étiquetage 28 comprend un circuit d'électrodes propre à générer un champ électrique dans une goutte spécifiquement, avec des paramètres déterminés, de sorte à activer le réactif de signalisation 16 par chauffage, ou pour effectuer une lyse sélective d'une cellule ou d'un lysosome.

Le module d'injection 32 est, par exemple, propre à faire sortir l'émulsion 6 par le conduit de sortie de la chambre 20 pour qu'elle soit injectée dans le dispositif de récupération 30.

Le dispositif de récupération 30 est propre à permettre la récupération d'une goutte 4 ou d'une partie de la goutte en fonction de l'étiquetage. Par exemple le dispositif de récupération 30 permet la récupération de la goutte 4 entière. En variante, le dispositif de récupération 30 permet la récupération d'une cellule de la goutte 4. Par exemple le dispositif de récupération 30 comprend un cytomètre de flux ou un dispositif de tri. Un dispositif de tri utilisé, par exemple, est celui décrit dans l'article « Single-cell analysis and sorting using droplet-based microfluidics», publié en 2013 dans Nature Protocols vol 8, 870-891.

Le dispositif de récupération 30 est avantageusement apte à mesurer un signal correspondant au réactif de signalisation 16 activé et à effectuer un tri en fonction de ce signal.

Un premier procédé de sélection et de récupération de produits va maintenant être décrit. Le système de sélection et de récupération 1 précédemment décrit est fourni pour la mise en œuvre de ce procédé.

Le module d'injection 32 de l'émulsion 6 est fourni avec une émulsion 6 telle que précédemment décrite. L'émulsion 6 de gouttes 4 est injectée dans la chambre 22 au moyen du dispositif d'injection de l'émulsion 6. En variante, le système 1 est fourni directement avec l'émulsion 6 dans la chambre 22.

Avantageusement, dans la chambre 22, les gouttes 4 sont disposées selon un arrangement compact, notamment un arrangement en quinconce selon un plan transversal à l'axe Z au moins dans la région de mesure 38.

Lors d'une étape de mesure, un paramètre physique est mesuré pour plusieurs gouttes 4 de l'émulsion 6 présentes dans la région de mesure 38.

L'étape de mesure est effectuée dans la chambre 22 sans circulation des gouttes 4. La localisation spatiale 6 de chaque goutte est enregistrée en même temps que ces paramètres dans la mémoire du dispositif de classement 26.

Au cours de l'étape de mesure, le signal de fluorescence de la goutte 4 pour une longueur d'onde d'excitation donnée, et une longueur d'onde d'émission donnée est mesuré par le dispositif de mesure 24.

Par exemple, cette longueur d'onde correspond à la longueur d'onde d'un produit recherché.

A la suite de l'étape de mesure, le dispositif de classement 26 décide du classement des gouttes 4 en fonctions des mesures obtenues.

Par exemple, au cours de cette étape de classement, les résultats sont analysés et comparés entre eux et aux critères de classement. Le dispositif de classement 26 détermine pour au moins une goutte 4 à quelle classe elle appartient.

Au cours du classement, par exemple, toutes les gouttes 4 présentant une intensité, dans le canal de fluorescence du produit recherché, supérieure à un certain seuil sont attribuées à la même classe. Le seuil est par exemple déterminé par rapport à l'intensité moyenne de l'ensemble des gouttes 4 de l'émulsion 6.

Le classement est avantageusement automatisé. En variante, le classement est effectué par un opérateur au moyen d'une interface homme-machine.

Les gouttes 4 sont ensuite étiquetées au moyen du dispositif d'étiquetage 28 en fonction de leur classe.

Avantageusement, les gouttes 4 sont maintenues immobiles dans la chambre 22, le temps des étapes de mesure et d'étiquetage.

L'étiquetage comprend par exemple une étape d'illumination des gouttes 4 pour activer optiquement le réactif de signalisation 16. L'illumination est par exemple réalisée par un laser du dispositif d'étiquetage 28.

A la suite de l'étape d'étiquetage, une étape de récupération est effectuée. L'étape de récupération comprend une étape de transfert des gouttes 4 ou de leur contenu dans le dispositif de récupération 30. Par exemple, l'émulsion 6 est injectée dans le dispositif de récupération au moyen du module d'injection 32.

A la suite de l'étiquetage, les gouttes 4 qui ont été classées puis étiquetées peuvent être distinguées des autres gouttes, en fonction de leur marquage. Ainsi même si la répartition spatiale des gouttes 4 est modifiée, par exemple lors de leur collection ou de leur transfert vers le dispositif de récupération 30, il est possible de connaitre leur classe.

A la suite du transfert, une étape de mesure est avantageusement mise en œuvre par le dispositif de récupération 30 et toutes les gouttes 4 présentant un signal du réactif de signalisation 16 supérieur à un seuil de détection sont collectées dans un même tube. Le signal du réactif de signalisation 16 activé permet de récupérer spécifiquement ces gouttes 4 lors d'une étape de récupération. Les gouttes 4 sont triées en fonction de leur étiquetage.

Un tel système permet ainsi la séparation de l'émulsion 6 en au moins deux populations de gouttes et la récupération spécifique d'une population de gouttes 4.

Le maintien des gouttes 4 dans la zone de mesure 38 pendant les étapes de mesure et d'étiquetage permet d'obtenir une caractérisation fine et précise des gouttes 4 étiquetées. En outre, l'étape de mesure peut être plus longue que dans des systèmes de cytométrie en flux. La mesure peut par exemple être réitérée. Plusieurs mesures peuvent ainsi être intégrées par goutte 4 avant d'effectuer l'étape de classement. Ceci évite de sélectionner une goutte 4 sur la base d'une seule mesure qui peut être bruitée. Les gouttes 4 classées sont ainsi mieux caractérisées avant leur récupération.

En outre, comme la récupération se fonde sur le signal d'étiquetage et non sur le signal de mesure. Il est possible d'isoler efficacement une classe de gouttes 4 qui présentent des caractéristiques mesurées proches des autres gouttes 4 en ajustant précisément les critères de classement avant l'étiquetage.

Un premier exemple de mise en œuvre du premier procédé va maintenant être décrit en regard des figures 3 et 4. Dans cet exemple, l'émulsion 6 est formée à partir de trois émulsions comprenant des gouttes comprenant différentes concentrations de sulforhodamine. Chaque goutte 4 de chaque émulsion comprend, en outre, la même proportion du réactif de signalisation 16 qui est de la fluorescéine en cage CMNB.

La concentration du réactif de signalisation 16 utilisé pour l'étiquetage est, par exemple, comprise entre 1 nM et 100 µM et avantageusement égale à 2 µM.

Au cours de l'étape de mesure, le signal de fluorescence correspondant à la sulforhodamine est enregistré pour plusieurs gouttes 4 de la région de mesure 38. La figure 3 représente une partie de la région de mesure 38 observéef dans le canal de fluorescence correspondant à la sulforhodamine. Sur cette figure, il est possible de distinguer trois types de gouttes 4 présentant respectivement une intensité faible 60, une intensité moyenne 62 ou une intensité forte 64 du signal de fluorescence. Chaque type de gouttes 60, 62, 64 provient dans cet exemple d'une émulsion avec une concentration de sulforhodamine différente. Les gouttes 64 présentant la plus forte intensité sont les gouttes 4 comprenant la plus forte concentration de sulforhodamine.

Au cours du classement, l'intensité moyenne de l'ensemble des gouttes 4 de l'émulsion est calculée. Un seuil d'intensité est calculé comme étant, par exemple, 1,5 fois cette valeur moyenne. Toutes les gouttes 4 présentant une intensité, dans le canal de fluorescence du produit recherché, supérieure à ce seuil sont attribuées à une première classe. Ces gouttes 4 classées dans la première classe correspondent aux gouttes 64 d'intensité forte sur la figure 3.

Au cours de l'étape d'étiquetage, les gouttes 64 appartenant à la première classe sont éclairées sélectivement par un flash UV, à 355 nm, présentant une durée de 5 secondes. Avec ces flashs, les cages de CMNB sont clivées et la fluorescéine est libérée. Le réactif de signalisation 16 de ces gouttes 4 est ainsi activé.

La figure 4 représente la même partie de la région de mesure 38 que la figure 3, après l'étape d'étiquetage, observée dans le canal de fluorescence correspondant à la fluorescéine. Seules les gouttes 64 qui présentaient une forte intensité sur la figure 3, ont été marquées lors de l'étape d'étiquetage. Elles présentent ainsi avec une intensité importante dans le canal de la fluorescéine. Le contraste est important car les autres gouttes 60, 62, non classées, n'ont pas été illuminées par les flashs UV, leur réactif de signalisation 16 n'a pas été activé, elles ne sont donc pas fluorescente dans le canal de fluorescence de la fluorescéine. Cette figure montre que les gouttes 64 classées ont été étiquetées mais pas les autres.

Au cours de l'étape de récupération, les gouttes 4 sont triées en fonction de leur signal de fluorescence dans un canal correspondant à la fluorescéine par le dispositif de récupération 30. Les gouttes 64 qui présentaient une forte concentration de sulforhodamine sont ainsi isolées des autres gouttes 60, 62, de l'émulsion 6 sur la base d'un signal d'étiquetage présentant un fort contraste. Elles peuvent ainsi être récupérées efficacement et rapidement.

Un deuxième procédé de sélection et de récupération de produits va maintenant être décrit. Ce procédé diffère du premier procédé en ce que au cours de l'étape de classement, une pluralité de gouttes 4 sont classées lors de l'étape de classement dans une pluralité de classes.

Au cours de l'étape d'étiquetage, chaque goutte 4 d'une classe est illuminée avec des conditions d'illumination déterminées, le niveau du signal de fluorescence émis par le réactif de signalisation 16 dépendant des conditions d'illumination lors de l'activation. Les gouttes 4 sont ensuite triées en fonction de leur niveau de fluorescence lors de l'étape de récupération.

Dans un deuxième exemple, le deuxième procédé est mis en œuvre. Le deuxième exemple est identique au premier exemple jusqu'à l'étape de classement. Au cours de l'étape de classement, deux seuils sont déterminés. Un premier seuil d'intensité est calculé comme étant 1,5 fois cette valeur moyenne et un deuxième seuil est calculé comme étant 0,5 fois la valeur moyenne. Toutes les gouttes présentant une intensité supérieure au premier seuil sont attribuées à une première classe et toutes les gouttes présentant une intensité inférieure au deuxième seuil sont attribuées à une deuxième classe.

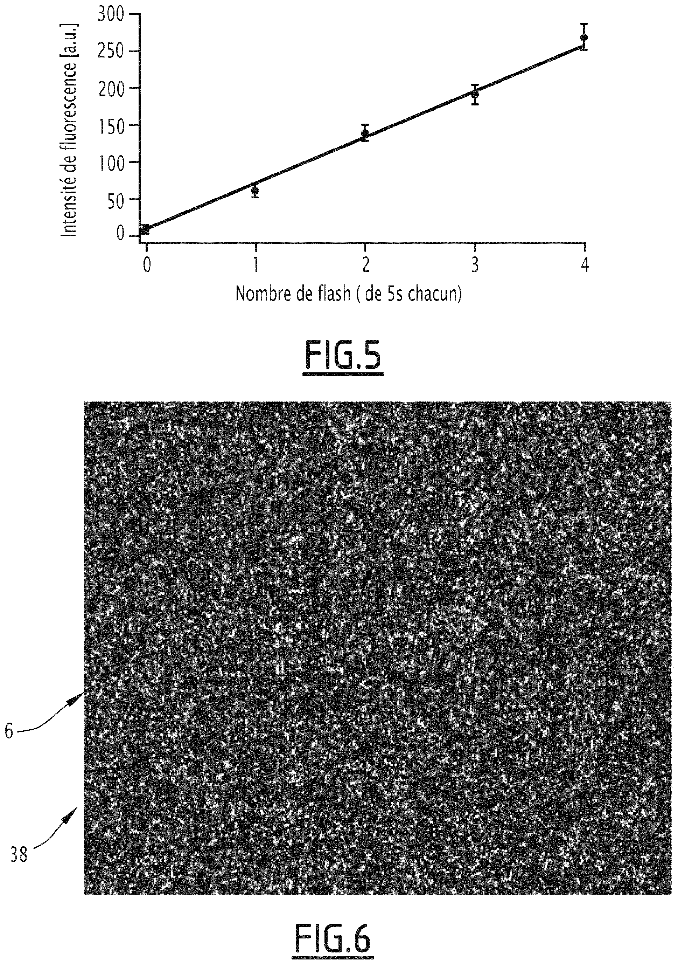

Plusieurs niveaux de fluorescence peuvent être atteints à partir de la même quantité de fluorescéine en cage CMNB, en modifiant le nombre de flashs UV envoyés sur une goutte 4 comme cela est représenté sur le graphique de la figure 5. Ceci permet de modifier le signal d'étiquetage en fonction des classes de la goutte 4. Ainsi les gouttes 64 de la première classe sont illuminées avec un flash. Les gouttes 60 de la deuxième classe sont illuminées avec quatre flashs.

A la suite de l'étape d'étiquetage, les gouttes 60 de la deuxième classe présentent une intensité forte dans le canal de la fluorescéine et les gouttes 64 présentent une intensité dans le canal de la fluorescéine plus faible mais facile à détecter. Les gouttes 62, n'ayant pas été classée ne sont pas fluorescentes dans le canal de la fluorescéine.

Au cours de l'étape de récupération, les gouttes 64 qui comprennent une forte concentration de sulforhodamine sont ainsi isolées. De même les gouttes 60 qui comprennent une faible concentration de suflorhodamine sont isolées sur la base d'un signal d'étiquetage présentant un fort contraste. Chaque catégorie peut ainsi être récupérée efficacement.

Un troisième procédé de sélection et de récupération de produits va maintenant être décrit. Ce procédé diffère du premier procédé et du deuxième procédé en ce que la base commune 12 de chaque goutte 4 comprend en outre un ou plusieurs réactifs de test.

Les réactifs de test permettent la mesure de paramètres physique dans la goutte 4. Chaque réactif de test est présent dans les mêmes proportions dans chaque goutte 4 d'une émulsion 6.

Par exemple, les réactifs de test sont les réactifs nécessaires à la réalisation d'un test ELISA en goutte ou sont les réactifs d'un test immunologique.

Avantageusement, au moins un réactif de test ou un produit des réactions de test est fluorescent. Des mesures sont alors effectuées par le dispositif de mesure 24 sur différents canaux de fluorescence correspondant aux différents réactifs ou produits fluorescent.

Le réactif de signalisation 16 est avantageusement choisi pour ne pas recouper les canaux de fluorescence des réactifs ou produits du test.

Au cours de l'étape de mesure, l'efficacité des éléments propres 14 des gouttes est testée et mesurée par les tests.

Avantageusement, l'étape de mesure comprend une mesure d'un paramètre caractéristique du test, réitérée à différents instant successifs. La réitération de la mesure permet d'effectuer un suivi cinétique de la réaction de test au sein des gouttes 4.

Dans le troisième exemple représenté sur la figure 6, la région de mesure 38 présente une longueur de 10 mm et une largeur de 8 mm et comprend 40000 gouttes. Les différents points correspondent chacun à une goutte et les différents niveaux de gris correspondent à des intensités de fluorescence différentes dans un canal d'excitation correspondant à un réactif de test. La durée de l'étape de mesure dépend des tests effectués. Dans l'exemple de la figure 6, une mesure sur quatre canaux de fluorescence différents pour 40000 gouttes dure environ 10 minutes.

Une fois les mesures effectuées, il est possible d'effectuer un classement des gouttes selon les résultats. Le classement porte ainsi sur plusieurs paramètres mesurés. Les gouttes 4 peuvent ainsi être classées en fonction des résultats aux tests. Par exemple, les gouttes 4 sont classées selon les rendements des réactions qui ont lieu dans les gouttes 4 et selon les vitesses de ces réactions.

Par exemple, les gouttes 4 qui présentent un bon résultat au test d'affinité d'un antigène sont repérées au cours de l'étape de classement. Elles sont classées dans une classe associée à leur résultat au test.

L'étiquetage de ces gouttes permet de pouvoir les séparer facilement des autres à l'aide d'un trieur classique. Les gouttes peuvent ainsi être récupérées facilement avec des signaux d'étiquetage correspondant à leur catégorie.

Ce procédé permet une récupération des gouttes simple car effectuée sur un seul paramètre, alors même que leur sélection est complexe et basée sur plusieurs paramètres. Comme la récupération est simple, les gouttes récupérées peuvent être analysées en aval par d'autres méthodes. Par exemple, si les gouttes contiennent des cellules, elles peuvent être séquencées ou remises en culture.

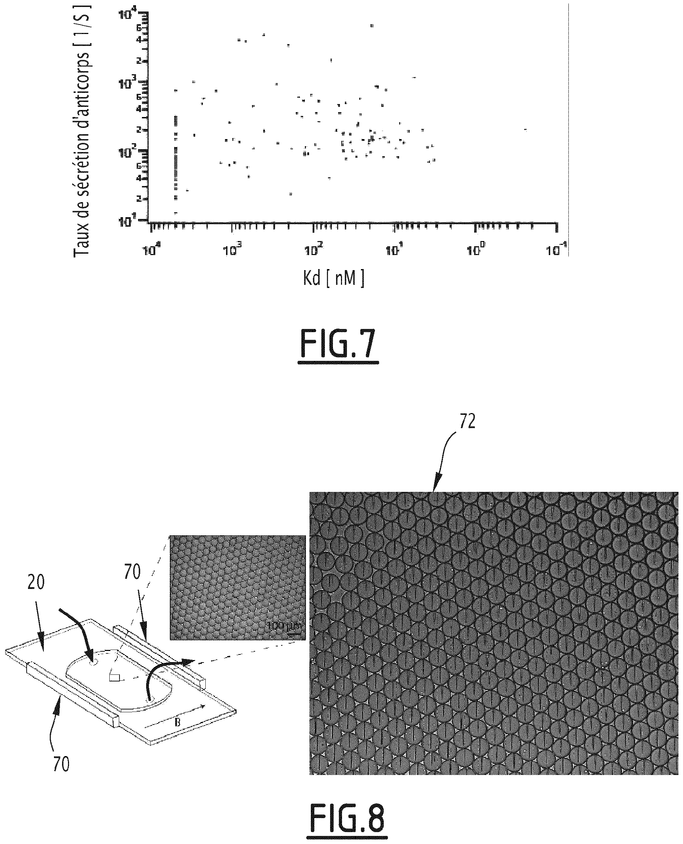

Un quatrième exemple illustrant la mise en œuvre du troisième procédé va maintenant être décrit en regard de la figure 7. Dans cet exemple, l'objectif est de repérer et d'isoler des cellules productrices d'un anticorps réagissant contre un antigène la protéine *Tetanus Toxoid* (TT).

Une émulsion 6 comprenant des gouttes de 40 pL et comprenant zero ou une cellule productrice d'anticorps est formée. Chaque goutte comprend des réactifs permettant la caractérisation phénotypique des anticorps produits, notamment des réactifs de test au TT. De plus, chaque goutte 4 comprend d'un marqueur photo-activable comme la fluorescéine en cage.

Les gouttes 4 sont disposées dans la chambre 22 selon un réseau à deux dimensions adapté pour que chaque goutte 4 de la zone de mesure 38 puisse être illuminée et visualisée séparément.

Au cours de l'étape de mesure, l'affinité et le nombre des anticorps est mesuré. La figure 7 représente pour chaque goutte 4, le taux de sécrétion d'anticorps en fonction de l'affinité des anticorps mesurée. Dans ce graphique, chaque point correspond à une goutte 4. Un tel graphique est par exemple affiché sur le dispositif de classement 26 pour permettre à l'utilisateur de choisir les critères de classement.

Dans cet exemple, 15 000 gouttes sont présentes dans la région de mesure 38, 12 000 gouttes comprennent des cellules. Parmi ces 12 000 gouttes, seuls 200 gouttes présentent des anticorps agissant contre le TT en quantité détectable, dont seulement 21 présentent une affinité élevée. Ces 21 gouttes sont placées dans une première classe à récupérer. Avantageusement, les autres gouttes comprenant des anticorps avec une affinité faible sont classées dans une deuxième classe pour servir de comparaison. De plus, quelques gouttes comprenant des cellules n'ayant pas généré d'anticorps et quelques gouttes ne comprenant pas de cellules sont classées dans une troisième classe et une quatrième classe pour servir, par la suite, comme blanc ou témoin négatif dans d'autres expériences permettant de caractériser les cellules.

L'illumination d'une goutte classée permet l'activation du marqueur photo-activable spécifiquement dans cette goutte, les gouttes 4 voisines n'étant pas illuminées. Au cours de l'étape d'étiquetage, les gouttes 4 sélectionnées sont illuminées une à une avec des paramètres correspondant à leur classe.

Au cours de l'étape de récupération, les gouttes 4 comprenant le plus d'anticorps ou les meilleurs anticorps sont récupérées sélectivement dans un tube, et les cellules, ayant sécrété ces anticorps peuvent être remises en culture. En variante à la suite de la récupération, les cellules sont lysées et leur ADN ou leur ARN est amplifié et séquencé par des techniques connues.

Le système permet de ne pas noyer ces cellules qui sont peu nombreuses dans un bruit de mesure lié aux nombreuses cellules des autres classes.

Un quatrième procédé de sélection et de récupération de produits va maintenant être décrit. Le réactif de signalisation 16 comprend un marqueur cellulaire à activer. Les gouttes classées comprennent une cellule. Le marqueur cellulaire une fois activé est propre à se fixer et à marquer la membrane des cellules présentes dans la goutte classée. Par exemple, le marqueur cellulaire activé fixé à la membrane est fluorescent.

L'étape d'étiquetage comprend un marquage de la cellule, et l'étape de récupération sélective comprend une rupture d'émulsion suivie d'un tri par cytométrie.

Par exemple, le marquage de la cellule est membranaire, intracellulaire, ou au niveau des organites cellulaire comme les lysosomes.

Un tel signal permet de récupérer spécifiquement les cellules étiquetées lors d'une étape de récupération. Au cours de cette étape de récupération, l'émulsion 6 est cassée de sorte à ce que les phases internes 8 des gouttes 4 fusionnent et forment une phase continue. La phase continue est récupérée. Un tri par cytométrie de la phase continue, par exemple dans un trieur de cellules par fluorescence, permet d'isoler les cellules marquées. Les cellules de la phase continue sont triées en fonction de leur marquage effectué au cours de l'étape d'étiquetage.

Un tel procédé permet la récupération spécifique d'une cellule après une mesure complexe de son phénotype pour de nombreuses cellules.

Un cinquième exemple illustrant la mise en œuvre du quatrième procédé va maintenant être décrit. Dans cet exemple, l'objectif est d'étiqueter directement les cellules au cours de l'étape d'étiquetage avant leur récupération par rupture de l'émulsion.

Par exemple, avant l'introduction dans les gouttes, les cellules sont marquées avec un réactif non-fluorescent qui réagit avec un élément de la membrane accessible, comme des groupes d'amines présents dans les protéines et certains lipides. Le réactif le plus favorable est activé par la lumière, et, dans ce processus, devient fluorescent. Le réactif est par exemple la carboxyfluorescéine en cage CMND, SE (5-Carboxyfluorescéine-Bis-(5-Carboxymethoxy-2-Nitrobenzyl) Ether, β-Alanine-Carboxamide, Succinimidyl Ester) commercialisée par ThermoFisher. L'activation en utilisant la lumière UV donne des cellules fluorescentes triables après la récupération des gouttes et des cellules.

Alternativement, les cellules sont marquées avant l'expérience avec du sulfo-SBED (Sulfo-N-hydroxysuccinimidyl-2-(6-[biotinamido]-2-(p-azido-benzamido)-exanoamido) ethyl-1,3'-dithioproprionate, Thermo Fisher 33033). Ce complexe comprend trois groupes réactionnels : un ester Sulfo-N-hydroxysuccinimidyl propre à réagir avec des groupes amines primaires, un groupe photo-activable (groupe azido-benzamide) et une molécule de biotine. Ce marqueur est ajouté à toutes les cellules avant l'expérience. Il réagit avec les groupes amines de la membrane de la cellule, présent dans des protéines ou des lipides membranaires.

Une émulsion 6 comprenant des gouttes de 40 pL et comprenant zero ou une de ces cellules fonctionnalisées avec le sulfo-SBED est formée. Les gouttes sont disposées dans la chambre 22 comme précédemment.

Suite à l'étape de mesure et à l'étape de classement, les cellules des gouttes sélectionnées sont illuminées par de la lumière UV.

La lumière UV active le groupe d'azido-benzamide qui réagit avec la membrane cellulaire formant une molécule attachée par deux groupes chimiques à la surface cellulaire. Après l'étiquetage, les gouttes sont récupérées et l'émulsion est cassée.

Une étape de centrifugation permet la purification des cellules. La récupération de toutes les cellules facilite la manipulation des cellules et aboutit à moins de perte des cellules d'intérêt.

Ensuite, les cellules sont mises à incuber dans une solution comprenant un agent réducteur, tel que du di-thiotreitol (DTT) à 25 mM. Ceci permet de cliver les chaines d'hydroxysuccinimidyl qui lient le groupe disulfide à la biotine. Ainsi, la biotine est supprimée de toutes les cellules qui n'ont pas été activées par les UV au cours de l'étape d'étiquetage. Dans les cellules étiquetées, la liaison du groupe disulfide est également clivée mais l'azido-benzamide activée retient la biotine à la membrane cellulaire.

En conséquence, seules les cellules illuminées au cours de l'étape d'étiquetage présentent des molécules de biotine à leur membrane.

La biotine peut ensuite être marquée par de la streptavidine fluorescente, telle que de la streptavidin-Alexa555, commercialisée par ThermoFisher. Les cellules marquées sont ainsi fortement fluorescentes par rapport au reste de la population de cellules. Les cellules peuvent ensuite être récupérées par FACS.

En variante, les cellules biotinylées sont liées à des billes magnétiques fonctionnalisées avec de la streptavidine. La purification des cellules est effectuée en les extrayant de la solution par voie magnétique.

En variante, avant la formation de l'émulsion, les cellules sont fonctionnalisées avec un autre marqueur membranaire activable présentant un groupe fluorescent ou un groupe chimique particulier tel que de l'azide, de la biotine, du NTA ou autre.

Dans une variante, le réactif de signalisation 16 comprend des amorces ou des réactifs permettant le marquage de l'ADN ou d'ARN à activer, par exemple le marquage d'ARN messagers. En outre les cellules sont par exemple lysées dans les gouttes 4 respectives.

Ce réactif de signalisation 16, une fois activé, est propre à marquer l'ADN ou l'ARN messager présent dans la goutte 4 sélectionnée, par exemple par un signal fluorescent.

En variante, l'ADN ou l'ARN d'intérêt est marqué par un groupe chimique comme un azoture, une alcyne, des chélateurs comme l'acide nitrilotriacétique (NTA), ou des particules magnétiques. Un tel marquage permet de récupérer spécifiquement les ADN, ARN, ou ARNm étiquetés lors d'une étape de récupération. Au cours de cette étape de récupération, l'émulsion est cassée de sorte à ce que les phases internes des gouttes forment une seule phase continue.

Les ADN ou ARN marqués sont purifiés en utilisant une méthode de purification approprié au réactif de signalisation 16 utilisé. Par exemple, ils sont récupérés par chimie click pour les azoture ou alcynes, par purification par résine de Nickel pour le NTA.

Une telle technique facilite, par exemple, le séquençage des ARNm intéressants.

Dans une variante, la goutte 4 ne comprend pas de réactif de signalisation 16. L'étape d'étiquetage consiste à effectuer une lyse laser des cellules de gouttes 4 classées. Lors de l'étape de récupération, l'émulsion 6 est cassée, et les produits sont purifiés par centrifugation. Les cellules non lysées et les débris sont retirés. Ceci permet de récupérer spécifiquement les cytoplasmes des cellules sélectionnées.

Une variante consiste à lyser toutes les cellules des gouttes non marquées. Lors de l'étape de récupération, l'émulsion 6 est cassée, les cellules lysées et les débris sont retirés. Ceci permet de récupérer spécifiquement les cellules sélectionnées.

Dans une variante, l'activation du réactif de signalisation 16 augmente sa densité. Au cours de l'étape d'étiquetage, la densité de la goutte 4 classée est augmentée. Au cours de l'étape de récupération l'émulsion est injectée sans être cassée dans un dispositif permettant la séparation par densité, par exemple dans une ampoule de décantation. Ceci permet une récupération des gouttes 4 classées.

Dans une variante, le réactif de signalisation 16 est propre à être activé sous des conditions de chauffage local. Le chauffage local est utilisé pour la lyse de cellules ou des liposomes. Les liposomes envisagés libèrent une molécule, avantageusement qui permet de marquer la goutte ou la cellule après la libération. Cette molécule libérée peut entrainer un marquage fluorescent de la cellule ou de la goutte ou changer la nature de la cellule.

L'étape d'étiquetage comprend une étape d'activation thermique du réactif de signalisation 16 selon le classement de la goutte 4. Au cours de l'étape d'étiquetage, les gouttes sélectionnées sont chauffées localement au moyen du dispositif d'étiquetage.

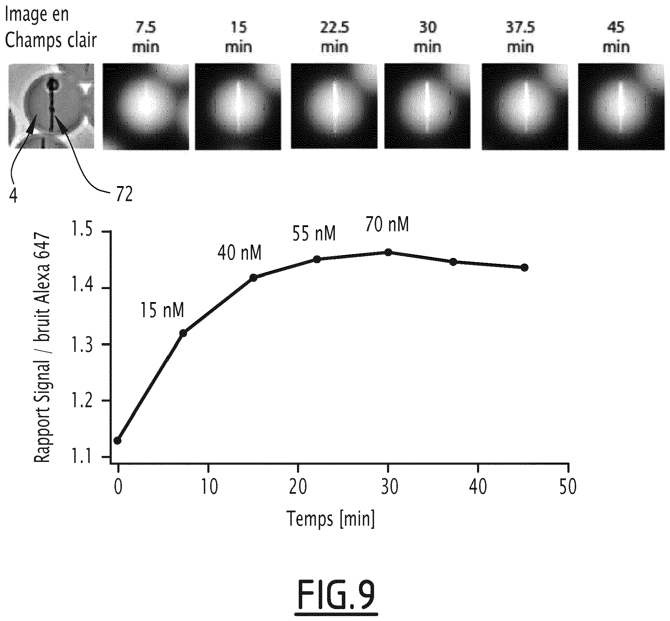

Un cinquième procédé va maintenant être décrit. Ce procédé est mis en œuvre dans le cinquième exemple représenté sur les figures 8 et 9.

Ce procédé diffère des procédés précédemment cités en ce que les gouttes comprennent au moins un agrégat de particules définissant un objet allongé selon un axe principal, au moins certaines gouttes contenant au moins un élément cible propre à se fixer sur l'agrégat, l'étape de mesure comprend la mesure d'un paramètre physique caractéristique de la fixation de l'élément cible sur l'agrégat.

Le support 20, tel qu'illustré sur la figure 8, comprend des dispositifs d'alimentation 70 propres à former un champ magnétique B pour que les particules forment un agrégat allongé 72.

Les particules sont des particules superparamagnétiques. Les particules sont propres à former un agrégat allongé 72 sous une forme de colonne sous l'application d'un champ magnétique. La mesure du paramètre physique caractéristique de la fixation de l'élément cible sur l'agrégat est effectuée localement en une pluralité de points situés dans la goutte, l'étape de mesure comportant de préférence la détermination de l'intégrale des valeurs mesurées au sein de la goutte. Au cours de l'étape de mesure, les particules sont organisées pour former l'agrégat allongé 72 en colonne. Ce système permet d'obtenir des mesures de fluorescence avec un meilleur contraste pour un suivi cinétique.

Le cinquième exemple illustre la possibilité de mesurer simultanément la cinétique de sécrétion et l'affinité pour un antigène donné, d'anticorps monoclonaux générés par des cellules primaires unique isolées en gouttes au cours de l'étape de mesure. L'élément propre 14 permettant la sélection de la goutte 4 est un anticorps monoclonal sécrété par une cellule primaire. Cette sécrétion est mesurée de façon cinétique.

Dans cet exemple, les gouttes présentent un volume de 40 picolitres. Dans cet exemple, les cellules à analyser et à classer sont des lymphocytes B préalablement extraits de la rate d'une souris et purifiés suivant une procédure standard (Pan B kit II #130-104-443, Miltenyi Biotec).

Les réactifs de test comprennent un antigène, une entité de quantification et des particules propres à former un agrégat 72 et présentant une entité de capture. L'antigène est apte à former un complexe avec l'anticorps recherché. L'entité de quantification est également apte à former un complexe avec l'anticorps.

L'antigène est un antigène fluorescent, plus précisément « TT-AF488 » la protéine *Tetanus Toxoid* préalablement fonctionnalisée avec un fluorophore AlexaFluor-488, grâce à une procédure de marquage d'un kit standard par exemple fourni par ThermoFisher. Chaque goutte comprend 25 nM de TT-AF488. Les particules magnétiques utilisées pour former les colonnes en gouttes sont marquées à saturation avec une entité de capture, ici le « CaptureSelectTM Biotin Anti-LC-kappa (Murine) conjugate » (ThermoFisher #7103152500). Ces particules sont des particules magnétique Streptavidine (Ademtech #0433), marquées à saturation avec l'entité de capture. Dans l'exemple, l'entité de quantification utilisée est le Rabbit Fab'2 anti-FCmouse AF647, JacksonIR #315-606-146. Chaque goutte comprend 75nM de cette entité de quantification.

Le ratio du signal de fluorescence de l'antigène sur le signal de fluorescence de l'entité de quantification est corrélé à la constante de dissociation de l'anticorps. La constante de dissociation Kd est évaluée à partir de la mesure de concentration d'antigène fluorescent lié à l'anticorps immobilisé sur la colonne de billes et de la mesure simultanée de la concentration d'anticorps capturée sur la colonne de billes.

La figure 9 est un graphique représentant l'évolution, à l'échelle de la cellule unique, du ratio du signal relocalisé de la ligne de billes magnétiques sur le signal dispersé de la goutte en fonction du temps (en minutes). Par « signal relocalisé », on entend le signal de fluorescence à proximité de l'agrégat et par « signal dispersé », on entend le signal de fluorescence dans le reste de la goutte.

De plus, grâce à une courbe de titration, il est possible de corréler le signal mesuré à une concentration d'anticorps monoclonaux. Cette concentration est notée sur la figure 9. Les courbes de titration sont par exemple réalisées avec le dispositif de mesure 24 à partir d'émulsions comprenant une quantité connue d'anticorps anti-TT, dit TT7. Le TT7 utilisé pour la réalisation de courbes de titration a été obtenu par expression de protéine recombinante à partir de la séquence publiée dans l'article écrit par Brandon J DeKosky et al., intitulé « High-throughput sequencing of the paired human immunoglobulin heavy and light chain repertoire » paru dans Nature Biotechnology Volume:31, pages 166 à 169 en 2013.

Le procédé de sélection et de récupération de produits selon l'invention est plus fiable et plus rapide que les procédés existants, et permet une caractérisation précise et une récupération efficace du contenu intéressant d'une goutte.

Un avantage du procédé est qu'il permet d'effectuer des mesures cinétiques avant la récupération. Ceci permet le suivi de réponse biologique à des stimuli et permet de calculer différents paramètres. En outre, le fait de pouvoir effectuer plusieurs mesures permet de mieux distinguer les faux positifs des positifs à un test.

Ce système 1 est utile pour étudier les systèmes cellulaires au niveau des cellules uniques. En outre, ce système permet une sélection basée sur des phénotypes complexes. Par exemple, des interactions entre des cellules et des bactéries peuvent être observées au cours de l'étape de mesure. Ceci permettrait de classer et de récupérer les cellules dans différents groupes selon leur comportement vis-à-vis des bactéries. De même des interactions entre différentes cellules présentes dans une même goutte peuvent être observées au cours de l'étape de mesure.

Le système 1 permet la caractérisation de sécrétions cellulaires par goutte. Les sécrétions cellulaires analysées peuvent être des sécrétions d'anticorps, de cytokines et d'hormones. Si la goutte comprend une cellule, les sécrétions dans la goutte correspondent à cette cellule. Par conséquent, il est très avantageux de pouvoir mesurer des paramètres des sécrétions et de récupérer la goutte entière ou la cellule ayant conduit à ces sécrétions. Un tel système permet d'effectuer des études in vivo de cellules productrices d'anticorps en permettant d'une part une caractérisation fonctionnelle de leur phénotype, notamment par une détermination des coefficients de liaison, de l'affinité et de la spécificité des anticorps sécrétés avec un antigène cible et d'autre part une récupération des cellules. La récupération permet par exemple des études du génotype des cellules sélectionnées. Le fait de sélectionner les cellules en fonction de leur phénotype évite d'avoir à séquencer les cellules dont le phénotype n'est pas intéressant et permet de trouver plus facilement les séquences génétiques intéressantes. En effet, les corrélations sont plus faciles à faire et le bruit de mesure est réduit.

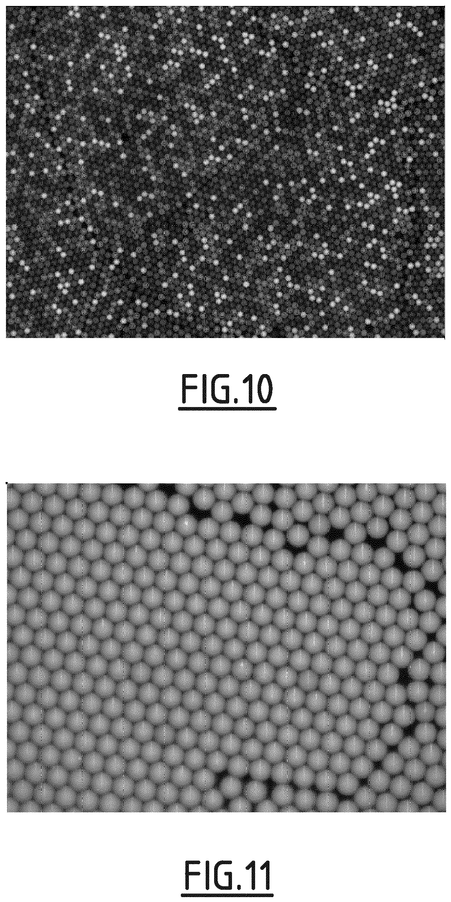

Les figures 10 et 11 illustrent un sixième exemple d'application du système de sélection et de récupération des produits. Ce sixième exemple est similaire au premier exemple.

La figure 10 représente une émulsion 6 formée à partir de gouttes comprenant différentes concentrations de sulforhodamine avant la sélection par le procédé selon l'invention.

Dans cet exemple, seules les gouttes comprenant une concentration de sulforhodamine de 200 nM sont étiquetées par libération de fluoroscéine en cage. Seules les gouttes étiquetées sont ensuite récupérées.

La figure 11 illustre une image des gouttes récupérées. Ces gouttes présentent sensiblement la même intensité.

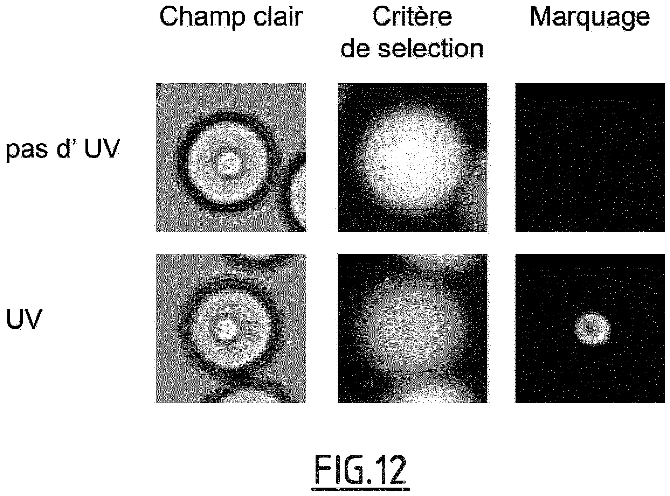

Un septième exemple va maintenant être décrit,en regard de la figure 12. La figure 12 illustre des cellules qui ont été marquées en utilisant de la carboxy fluoroscéine -NHS en cage. Dans cette expérience, le critère de sélection est un faible niveau de concentration de sulforhodamine. Seules les gouttes comprenant une concentration de sulforhodamine de 200 nM sont étiquetées. Les gouttes sélectionnées sont étiquetées par une exposition de la cellule aux UV.

Un huitième exemple va maintenant être décrit. Cet exemple illustre l'étiquetage de cellules avec de la biotine.

Dans ce huitième exemple, une solution de 10 cellules MIO par millilitre est mise en incubation avec 50 micro-M de sulfo-SBED pendant 15 minutes sur de la glace. Les cellules sont récupérées par centrifugation, rincées et resuspendues dans une solution tampon.

Les cellules sont séparées en deux lots. Le premier lot est exposé à des ultraviolets pendant 10 secondes. Le deuxième lot n'est pas exposé aux ultraviolets.

Ensuite, 5mM de DTT, un agent réducteur, est ajouté dans chaque lot et on laisse les solutions en incubations pendant 30 minutes à 4 degré Celsius.

Pour le deuxième lot pour lequel les cellules ne sont pas exposées aux UV, le réactif de signalisation est clivé.

Au contraire, dans le premier lot ayant reçu de la lumière UV, le réactif de signalisation se lie à la cellule par un groupe fonctionnel.

Ensuite, les cellules sont rincées deux fois et 300 nM de streptavidine-alexa 555 est ajoutée aux cellules.

On observe que la streptavidine-alexa 555 se lie et marque uniquement les cellules ayant été exposées aux UV.

Un neuvième exemple va maintenant être décrit. Cet exemple illustre une analyse en cytométrie des cellules marquées en fluorescence. Dans cette expérience, des cellules issues d'une lignée cellulaire sont prélevées à une concentration de 10 cellules par millilitre. Il est ajouté 100 microlitres d'une solution à 200 nM de fluoroscéine-NHS en cage. Les cellules sont ensuite mises en incubation pendant 30 minutes sur de la glace et ensuite encapsulées dans des gouttes. Un pourcentage, 11,34% de cellules, est exposé à la lumière UV et l'émulsion est retirée de la chambre, cassée puis les cellules sont purifiées. Seules les cellules ayant été exposées à la lumière UV sont étiquettées.

Afin de distinguer les cellules des débris cellulaires, les cellules sont mises en incubation avec un marqueur de vie cellulaire, de la calcéine rouge.

Avec l'analyse au cytomètre, on mesure que 9,43% des cellules positives sont vivantes et étiquetées.

Dans un autre exemple des cellules secrétant des IgG sont étiquetées après détection d'une sécrétion d'IgG.

## Revendications

1. Procédé de sélection et de récupération de produits comprenant les étapes suivantes :
- fourniture d'une émulsion (6) comprenant une pluralité de gouttes (4) contenues dans un fluide porteur (10), chaque goutte comprenant un fluide interne (8),
- mesure d'au moins un paramètre physique pour plusieurs gouttes (4) de l'émulsion (6),
- classement d'au moins une partie des gouttes (4) de l'émulsion dans une classe en fonction des mesures obtenues lors de l'étape de mesure,
- étiquetage d'au moins une partie des gouttes (4) classées en fonction de la classe de la goutte (4), et
- récupération sélective de la goutte (4) ou d'une partie de la goutte (4) en utilisant l'étiquetage de la goutte ou d'une partie de la goutte (4), le fluide interne (8) de chaque goutte (4) comprenant un réactif de signalisation (16), l'étape d'étiquetage comprenant une étape d'activation optique du réactif de signalisation (16) selon le classement de la goutte (4).

2. Procédé selon la revendication 1, dans lequel une pluralité de gouttes (4) sont classées simultanément lors de l'étape de classement et l'étape de récupération comporte un tri des gouttes (4) après l'étiquetage de chaque goutte (4) classée.

3. Procédé selon l'une quelconque des revendications 1 ou 2, dans lequel l'étape de mesure comprend une mesure d'au moins un paramètre réitérée à différents instants successifs sur les mêmes gouttes, de préférence pour un suivi cinétique de réaction au sein de chaque goutte (4).

4. Procédé selon l'une quelconque des revendications précédentes dans lequel pour chaque goutte d'une pluralité de gouttes, l'étape de mesure comprend une mesure de plusieurs paramètres distincts sur la même goutte.

5. Procédé selon l'une quelconque des revendications précédentes, dans lequel une pluralité de gouttes (4) sont classées lors de l'étape de classement dans une pluralité de classes, l'étape d'étiquetage consistant à illuminer chaque goutte (4) d'une classe avec des conditions d'illumination déterminées, le réactif de signalisation (16) étant propre à émettre un signal lumineux, avantageusement de fluorescence après son activation, les gouttes (4) étant triées en fonction de leur niveau de signal lumineux émis lors de l'étape de récupération, le niveau du signal lumineux émis par le réactif de signalisation (16) dépendant des conditions d'illumination lors de l'activation.

6. Procédé selon l'une quelconque des revendications précédentes dans lequel le fluide interne (8) de chaque goutte (4) comprend un réactif de signalisation (16), l'étape d'étiquetage comprend une étape d'activation thermique du réactif de signalisation (16) selon le classement de la goutte.

7. Procédé selon l'une quelconque des revendications précédentes, dans lequel l'émulsion (6) est fournie dans une chambre bidimensionnelle (22), l'émulsion (6) étant maintenue sous forme d'une monocouche de gouttes (4) dans la chambre bidimensionnelle (22) au cours de l'étape de mesure et de l'étape d'étiquetage.

8. Procédé selon l'une quelconque des revendications précédentes dans lequel une goutte (4) classée comporte une cellule, l'étape d'étiquetage comprenant un marquage de la cellule, et l'étape de récupération sélective comprenant une rupture d'émulsion suivi d'un tri par cytométrie.

9. Système (1) de sélection et de récupération de produits comprenant :
- un support (20) propre à recevoir une émulsion (6) comprenant une pluralité de gouttes (4) contenues dans un fluide porteur (10), chaque goutte (4) comprenant un fluide interne (8),
- un dispositif de mesure (24) d'au moins un paramètre physique pour plusieurs gouttes (4) de l'émulsion (6) reçue dans le support (20),
- une unité de classement (26) propre à décider du classement d'au moins une goutte (4) de l'émulsion (6) en fonction des mesures obtenues lors de l'étape de mesure,
- un dispositif d'étiquetage (28) propre à étiqueter une goutte (4) ou une partie de la goutte (4) en fonction de la décision de classement de la goutte (4),
- un dispositif de récupération (30) de la goutte (4) ou d'une partie de la goutte (4) en fonction de l'étiquetage, le fluide interne (8) de chaque goutte comprenant un réactif de signalisation (16), le dispositif d'étiquetage (28) étant propre à réaliser une activation optique du réactif de signalisation (16) selon le classement de la goutte (4).

## Patentansprüche

1. Verfahren zur Selektion und Rückgewinnung von Stoffen, das die folgenden Schritte umfasst:
- Bereitstellen einer Emulsion (6), die eine Mehrzahl von in einem Trägerfluid (10) enthaltenen Tropfen umfasst, wobei jeder Tropfen ein inneres Fluid (8) umfasst,
- Messen mindestens eines physikalischen Parameters für mehrere Tropfen (4) der Emulsion (6),
- Klassifizieren mindestens eines Teils der Tropfen (4) der Emulsion in eine Klasse abhängig von den bei dem Meßschritt erhaltenen Messungen,
- Kennzeichnen mindestens eines Teils der klassifizierten Tropfen (4) abhängig von der Klasse des Tropfens (4), und
- selektives Rückgewinnen des Tropfens (4) oder eines Teils des Tropfens (4) unter Verwendung der Kennzeichnung des Tropfens oder eines Teils des Tropfens (4), wobei das innere Fluid (8) jedes Tropfens (4) ein Signalgebungsreagenz (16) umfasst und der Schritt des Kennzeichnens einen Schritt der optischen Aktivierung des Signalgebungsreagenz (16) gemäß der Klassifizierung des Tropfens (4) umfasst.

2. Verfahren nach Anspruch 1, bei der die Mehrzahl von Tropfen (4) gleichzeitig bei dem Klassifizierungsschritt klassifiziert wird und der Rückgewinnungsschritt eine Sortierung der Tropfen (4) nach dem Kennzeichnen jedes klassifizierten Tropfens (4) aufweist.

3. Verfahren nach einem beliebigen der Ansprüche 1 oder 2, bei dem der Meßschritt eine Messung von mindestens einem Parameter umfasst, die zu unterschiedlichen aufeinanderfolgenden Zeitpunkten an denselben Tropfen wiederholt wird, vorzugsweise für die kinetische Weiterverfolgung der Reaktionen in jedem Tropfen.

4. Verfahren nach einem beliebigen der vorhergehenden Ansprüche, bei dem für jeden Tropfen einer Mehrzahl von Tropfen der Meßschritt eine Messung von mehreren unterschiedlichen Parametern an demselben Tropfen umfasst.

5. Verfahren nach einem beliebigen der vorhergehenden Ansprüche, bei dem eine Mehrzahl von Tropfen (4) bei dem Klassifizierungsschritt in eine Mehrzahl von Klassen klassifiziert wird, wobei der Kennzeichnungsschritt darin besteht, jeden Tropfen (4) einer Klasse mit bestimmten Beleuchtungsbedingungen zu bestrahlen, wobei das Signalisierungsreagenz geeignet ist, ein Lichtsignal, vorzugsweise ein Fluoreszenzsignal nach seiner Aktivierung auszusenden, wobei die Tropfen (4) abhängig von dem Pegel des Lichtsignals, das während des Rückgewinnungsschritts ausgesandt wird, sortiert werden, wobei der Pegel des von dem Signalisierungsreagenz (16) emittierten Lichtsignals von den Beleuchtungsbedingungen bei der Aktivierung abhängt.

6. Verfahren nach einem beliebigen der vorhergehenden Ansprüche, bei dem das innere Fluid (8) jedes Tropfens (4) ein Signalisierungsreagenz (16) umfasst und der Kennzeichnungsschritt einen Schritt der thermischen Aktivierung des Signalisierungsreagenz (16) gemäß der Klassifizierung des Tropfens umfasst.

7. Verfahren nach einem beliebigen der vorhergehenden Ansprüche, bei dem die Emulsion (6) in eine zweidimensionale Kammer (22) geliefert wird, wobei die Emulsion (6) in Form einer einzigen Tropfenschicht in der zweidimensionalen Kammer (22) während des Meßschritts und des Kennzeichnungsschritts gehalten wird.

8. Verfahren nach einem beliebigen der vorhergehenden Ansprüche, bei dem ein klassifizierter Tropfen (4) eine Zelle aufweist, wobei der Kennzeichnungsschritt eine Markierung der Zelle umfasst und der Schritt der selektiven Rückgewinnung eine Brechung der Emulsion gefolgt von einer Sortierung durch Zytometrie umfasst.

9. System (1) zur Selektion und Rückgewinnung von Stoffen, umfassend:
- einen Träger (20), der geeignet ist, eine Emulsion (6) aufzunehmen, die eine Mehrzahl von in einem Trägerfluid (10) enthaltenen Tropfen (4) umfasst, wobei jeder Tropfen (4) ein inneres Fluid (8) aufweist,
- eine Vorrichtung (24) zum Messen mindestens eines physikalischen Parameters für mehrere Tropfen (4) der in dem Träger (20) aufgenommenen Emulsion (6),
- eine Klassifizierungseinheit (26), die geeignet ist, über die Klassifizierung mindestens eines Tropfens (4) der Emulsion (6) abhängig von den bei dem Meßschritt erhaltenen Messungen zu entscheiden,
- eine Kennzeichnungsvorrichtung (28), die geeignet ist, einen Tropfen (4) oder einen Teil des Tropfens (4) abhängig von der Entscheidung der Klassifizierung des Tropfens (4) zu kennzeichnen,
- eine Vorrichtung (30) zum Rückgewinnen des Tropfens (4) oder eines Teils des Tropfens (4) abhängig von der Kennzeichnung, wobei das innere Fluid (8) jedes Tropfens ein Signalisierungsreagenz (16) umfasst und die Kennzeichnungsvorrichtung (28) geeignet ist, eine optische Aktivierung des Signalisierungsreagenz (16) gemäß der Klassifizierung des Tropfens (4) zu realisieren.

## Claims

1. A method for selecting and recovering products comprising the following steps:
- providing an emulsion (6) comprising a plurality of drops (4) contained in a carrier fluid (10), each drop comprising an internal fluid (8),
- measuring at least one physical parameter for several drops (4) of the emulsion (6),
- classifying at least some of the drops (4) of the emulsion in a class based on measurements obtained during the measuring step,
- tagging at least some of the classified drops (4) based on the class of the drop (4), and
- selectively recovering the drop (4) or part of the drop (4) using the tag of the drop or part of the drop (4), the internal fluid (8) of each drop (4) comprising a signal reagent (16), the tagging step comprising a step for optical activation of the signal reagent (16) depending on the classification of the drop (4).

2. The method according to claim 1, wherein a plurality of drops (4) are classified simultaneously during the classification step and the recovery step includes sorting the drops (4) after tagging each classified drop (4).

3. The method according to any one of claims 1 or 2, wherein the measuring step comprises measuring at least one parameter reiterated at different successive moments on the same drops, preferably for a kinetic reaction monitoring within each drop (4).

4. The method according to any one of the preceding steps, wherein for each drop of a plurality of drops, the measuring step comprises measuring several distinct parameters on the same drop.

5. The method according to any one of the preceding claims, wherein a plurality of drops (4) are classified during the classification step in a plurality of classes, the tagging step consisting of illuminating each drop (4) from a class with preset illumination conditions, the signal reagent (16) being able to emit a light signal, advantageously fluorescence, after its activation, the drops (4) being sorted based on their light signal level emitted during the recovery step, the level of the light signal emitted by the signal reagent (16) depending on illumination conditions during the activation.

6. The method according to any one of the preceding claims, wherein the internal fluid (8) of each drop (4) comprises a signal reagent (16), the tagging step comprises a step for thermal activation of the signal reagent (16) depending on the classification of the drop.

7. The method according to any one of the preceding claims, wherein the emulsion (6) is provided in a two-dimensional chamber (22), the emulsion (6) being kept in the form of a single layer of drops (4) in the two-dimensional chamber (22) during the measuring step and the tagging step.

8. The method according to any one of the preceding claims, wherein a classified drop (4) includes a cell, the tagging step comprising marking the cell, and the selective recovery step comprising an emulsion break followed by sorting by cytometry.

9. A system (1) for selecting and recovering products comprising:
- a support (20) able to receive an emulsion (6) comprising a plurality of drops (4) contained in a carrier fluid (10), each drop (4) comprising an internal fluid (8),
- a device (24) for measuring at least one physical parameter for several drops (4) of the emulsion (6) received in the support (20),
- a classifying unit (26) able to decide the classification of at least one drop (4) of the emulsion (6) based on measurements obtained during the measuring step,
- a tagging device (28) able to tag a drop (4) or part of the drop (4) based on the classification decision of the drop (4),
- a recovery device (30) of the drop (4) or part of the drop (4) based on the tagging, the internal fluid (8) of each drop (4) comprising a signal reagent (16), the tagging device being able to achieve an optical activation of the signal reagent (16) depending on the classification of the drop (4).
